# EUROPÄISCHE PATENTSCHRIFT

(11) **EP 2 065 019 B1**
(45) Veröffentlichungstag und Bekanntmachung des Hinweises auf die Patenterteilung: **30.06.2010**
(21) Anmeldenummer: 07022933.1
(22) Anmeldetag: 27.11.2007
(51) Int. Cl.: A61F 5/058

(54) **Schiene zur Ruhigstellung eines Gelenks**
Splint for securing a joint
Attelle destinée à l'immobilisation d'une articulation

(43) Veröffentlichungstag der Anmeldung: 03.06.2009
(60) Teilanmeldung: 09011974.4
(73) Patentinhaber: Kandt, Olaf, 25474 Bönningstedt (DE)
(72) Erfinder: Kandt, Olaf, 25474 Bönningstedt (DE)
(74) Vertreter: Seemann, Ralph

(56) Entgegenhaltungen:
- US-A- 3 496 934
- US-A- 3 750 660
- US-A- 3 934 583
- US-A- 4 840 168
- US-A- 5 772 620
- US-A1- 2004 024 337
- US-B1- 6 293 918
- US-B1- 6 342 043

## Beschreibung

Die Erfindung betrifft eine Schiene zur Ruhigstellung eines Gelenks, die am ruhig zu stellenden Gelenk zu tragen ist, wobei die Schiene aus einem Flächenkörper besteht.

Bei Beanspruchung des Muskelapparats des menschlichen Körpers kommt es im Alltag, am Arbeitsplatz und in der Freizeit immer wieder zu Verletzungen, die es notwendig machen, dass diese unter Einschluss eines oder mehrerer Gelenke ruhig gestellt werden, um eine komplikationslose Ausheilung zu gewährleisten.

Verletzungen der Knochen, Sehnen, Muskeln, Nerven oder Blutgefäße gehen oftmals mit großen Schmerzen einher. Dies gilt insbesondere für Knochenbrüche und Weichteilverletzungen. Symptome hierbei sind Bewegungs-, Anspannungs-, Druck- und Dehnschmerzen sowie Blutungen. Besonders häufig sind Verletzungen im Breiten- und Leistungssport sowie im Alltag. Eine Vielzahl von in diesen Bereichen auftretenden Verletzungen und Schmerzzuständen erfordert eine Schienenversorgung.

Bei leichten Verletzungen und Überlastsyndromen sind neben dem Ruhigstellen auch Kälteanwendungen sowie das Hochlagern des betroffenen Körperteils üblich. Weiterhin kommen ruhig stellende Tapeverbände, Salbenverbände und Zinkleimverbände sowie physikalische Therapien, Orthesen sowie Bandagenversorgungen zum Einsatz.

Bei schweren Verletzungen oder Schmerzzuständen werden ausschließlich Schienenverbände angelegt. Dabei wird der betroffene Körperteil unter Einschluss eines oder mehrerer Gelenke durch Einsatz einer Schiene oder Fertigorthese ruhig gestellt. Üblicherweise werden hierzu Schienen aus Materialien wie Naturgips, thermoplastischem Kunststoff, Glasfasern oder Polyester sowie Fertigorthesen aus Kunststoff oder Aluminium verwendet.

Fertigorthesen und individuell anzufertigende Schienen unterscheiden sich darin, dass die Ruhigstellung eines oder mehrerer Gelenke nach dem Anlegen einer individuell anzufertigenden Schiene eine Fixierung mit einer Binde erfordert. Fertigorthesen werden dagegen mit Klettverschlusssystemen ausgestattet, die zur Fixierung dienen. Aufgrund der aufwendigeren Herstellung werden Fertigorthesen aus Kostengründen ausschließlich bei Langzeitversorgungen angewendet. Bei kurzfristigen Anwendungen kommen Schienen zum Einsatz, die vom Therapeuten selbst hergestellt und individuell angepasst werden.

Die Schienenversorgung ist zeitaufwendig und kostenintensiv, da eine individuelle Anfertigung und Anpassung notwendig ist. Wegen des hohen Materialgewichts in verarbeitungsbedingter scharfkantiger Formung sind solche Schienen oftmals mit einem schlechten Tragekomfort zu tragen. Das Anlegen und Herstellen solcher Schienenverbände, wie z.B. das Formen des Gipses in die medizinisch gewünschte Schiene ohne scharfe Kanten, erfordern besondere Fähigkeiten und Kenntnisse, welche gezielt ausgebildet und trainiert werden müssen.

Mit solchen bekannten Schienensystemen ist es nicht möglich, schnell am Ort einer gerade erlittenen Verletzung eine Schiene anzulegen, mit der das betroffene Gelenk bzw. Körperteil ruhig gestellt werden kann, um eine weitere Verschlimmerung der Verletzung kurzfristig zu verhindern. Auch sind solche Schienen nicht einsetzbar, wenn noch eine Versorgung von die Haut betreffenden Verletzungen notwendig ist, da sich herkömmliche Schienensysteme nicht flexibel abnehmen lassen, um danach wieder angebracht zu werden.

US 3 496 934 A betrifft einen wegwerfbaren Splint zur Ruhigstellung eines Körperteils, der aus einem Pappmaterial oder einem ähnlichen Material gefertigt ist. Dieser wird um einen Arm herumgelegt und mittels Knöpfen festgezurrt. Die Steifigkeit wird dem Material durch seine Stärke verliehen. Die Flexibilität erhält das Material durch im Wesentlichen parallele Falzlinien, die die Biegung bzw. Rollung des Materials um den Körperteil, beispielsweise einen Unterarm, lenken.

US 3 750 660 A betrifft ein Mittel zum Herstellen eines Splints, das ein flächiges Material, das um eine zentrale Falzlinie gefaltet wird und um das zu immobilisierende Körperteil herumgelegt wird. Dabei wird das Körperteil zwischen den beiden Flächen des Elements eingeschlossen und die überlappenden Flächen miteinander verbunden, beispielsweise verklebt. Das Material besteht beispielsweise aus Wellpappe.

Der Erfindung liegt daher die Aufgabe zugrunde, Schienen zur Ruhigstellung eines Gelenks, die am ruhig zu stellenden Gelenk zu tragen sind, zur Verfügung zu stellen, mit denen eine Sofortversorgung an einer Verletzung zur Ruhigstellung eines Gelenks erfolgen kann und die gleichzeitig einen erhöhten Tragekomfort bieten sowie kostengünstig herstellbar und konfektionierbar sind.

Diese Rufgabe wird durch eine Schiene zur Ruhigstellung eines Gelenks gemäß anabhängigern Anspruch 1 gelöst.

Die Außenkontur des Flächenkörpers ist so ausgebildet, dass sich im zusammengesetzten Zustand eine Schlaufe mit einer großen Öffnung zur Aufnahme von Teilen der an das ruhig zu stellende Gelenk angrenzenden Körperteile und einer der großen Öffnung gegenüber angeordneten kleinen Öffnung am ruhig zu stellenden Gelenk ergibt. Diese Schlaufenform mit verschieden großen Öffnungen ist geeignet für Schultergelenke, Ellenbogengelenke, Kniegelenke und Fußgelenke.

Unter einem Flächenkörper wird erfindungsgemäß ein flächiger Körper, wie beispielsweise ein Stück Karton oder Pappe, eine Kunststofffolie oder ähnliches, verstanden, der so geformt ist, dass durch Biegen oder Falten des Flächenkörpers eine geschlossene Schlaufe entsteht, in die das betroffene Gelenk bzw. die angrenzenden Körperteile eingelegt werden. Gegenüber einer aus reinem Verbandsmaterial geformten Schlaufe hat ein Flächenkörper eine höhere Steifigkeit und ist daher geeigneter zur Ruhigstellung eines Gelenks. Flexible Verbandsmaterialien wie Rollverbände sind daher keine Flächenkörper im Sinne der Erfindung.

Flächenkörper sind beliebig konfektionierbar und beanspruchen im zusammengefalteten Zustand extrem wenig Platz. Sie finden daher in Rettungswagen oder in Arzttaschen Platz, so dass auch eine Notfallversorgung oder Vorortversorgung von Verletzungen, die eine Ruhigstellung erfordern, möglich ist. Der Flächenkörper wird durch den Arzt in simpler Weise mittels eines Messers oder einer Schere an die Erfordernisse der ruhig zu stellenden Körperteile und der Größe der Person angepasst. Die Schlaufenform ist selbststabilisierend, so dass ein großer Materialaufwand nicht notwendig ist.

Vorteilhafterweise überlappen zwei Bereiche des Flächenkörpers, wobei die überlappenden Bereiche zur Bildung einer geschlossenen Schlaufe fest miteinander verbunden sind oder verbindbar sind. Die feste und flächige Verbindung der überlappenden Bereiche ist vorzugsweise eine Klebeverbindung, eine Steckverbindung oder eine Verbindung mittels Klammern, insbesondere Heftklammern. Auf diese Weise wird auf einfache und stabile Weise eine Schlaufe gebildet, die als Schiene zur Ruhigstellung eines Gelenks dient.

Wenn vorteilhafterweise der Flächenkörper wenigstens zwei Gruppen von Falzlinien mit jeweils wenigstens einer Falzlinie aufweist, wobei die Falzlinien jeder Gruppe untereinander parallel, aber zu den Falzlinien der anderen Gruppen unter einem Winkel angeordnet sind, ist eine Vorformung der erfindungsgemäßen Schiene bzw. Schlaufe gegeben. Vorteilhafterweise ist der Flächenkörper durch Biegung um die Falzlinien in die Form einer Schlaufe bringbar.

Vorzugsweise entspricht der Winkel zwischen den Falzlinien der verschiedenen Gruppen von Falzlinien dem Einstellwinkel für das ruhig zu stellende Gelenk. Da ein flächiges Material, das gebogen ist, in der Richtung, die auf die Biegung senkrecht steht, also entlang des Rückens der Biegung, sehr stabil ist, ist auf diese Weise sichergestellt, dass der Rücken der Biegung bzw. die Falzlinien, die die Biegung bestimmen, parallel zu den eingeschienten Extremitäten verlaufen. Dadurch weist die Schiene in dieser Richtung die größte Stabilität auf, ohne an anderer Stelle verstärkt werden zu müssen. Eine Verstärkung der belasteten Bereiche, beispielsweise durch zusätzliche Rippen entlang der am stärksten belasteten Stellen, ist ebenfalls vorteilhafterweise möglich.

Insbesondere vorteilhafterweise ist die Schiene in ihrer Kontur einem Schultergelenk, einem Ellenbogen, einem Kniegelenk oder einem Fußgelenk angepasst oder durch Zuschneiden anpassbar.

Durch die Anpassbarkeit wird eine einfache Herstellung ermöglicht, bei der die Schiene in einer größtmöglichen Länge hergestellt wird, so dass beispielsweise bei einer Schienung eines Ellenbogens immer der gesamte Ober- und Unterarm inklusive Handgelenk einfassbar sind. So ist es möglich, mit der erfindungsgemäßen Schiene in Schlaufenform zwei Gelenke gleichzeitig ruhig zu stellen.

Für die erfindungsgemäße Schiene in Form einer Schlaufe besteht in einer vorteilhaften Ausbildung der Flächenkörper aus einem zugfesten Material. Unter einem zugfesten Material wird im Rahmen der Erfindung ein Material verstanden, das gegenüber Zugbelastungen in der Fläche des Flächenkörpers resistent ist und nicht nachgibt. So erhalten die Schlaufe, insbesondere entlang der Biegungen bzw. Faltungen und Falzlinien ihre Festigkeit und Stabilität.

Eine besonders einfache Ausführungsform besteht darin, dass der Flächenkörper aus Pappe oder Karton bestehen, insbesondere aus Wellpappe. Diese Materialien sind in ihrer flächigen Ebene zugfest, jedoch um Falzlinien gut biegbar. Andere Materialien, wie Kunststoffe, die in einer Richtung senkrecht zu ihrer Fläche elastischer sind, können auch ohne Falzlinien gebogen werden.

In der Ausführungsform der Flächenkörper als Wellpappe sind die innen liegenden Rippen der Wellpappe vor-zugsweise wenigstens teilweise orthogonal zur Biegung des Flächenkörpers oder wenigstens teilweise parallel zu den Falzlinien des Flächenkörpers ausgerichtet. Damit sind die Rippen wenigstens teilweise entlang des Rückens einer Biegung des Flächenkörpers ausgerichtet und unterstützen die stabilisierende Wirkung der Biegung. Wellpappe ist in der Richtung der Rippen gegen Verbiegung besonders stabil.

Vorzugsweise ist die Schiene symmetrisch zur Anwendung an einem Gelenk sowohl eines linken als auch eines rechten Körperteils ausgebildet.

Die erfindungsgemäßen Schienen sind beispielsweise durch Umwickeln mit Verbandmaterial an den ruhig zu stellenden Gelenken bzw. den angrenzenden Körperteilen zu befestigen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung einer erfindungsgemäßen Schiene in flächiger Darstellung,
- Fig. 2: eine erfindungsgemäße Schiene in schematischer Seitenansicht,
- Fig. 3: eine erfindungsgemäße Schiene in schematischer Frontalansicht und
- Fig. 4: eine erfindungsgemäße Schiene in schematischer perspektivischer Darstellung.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

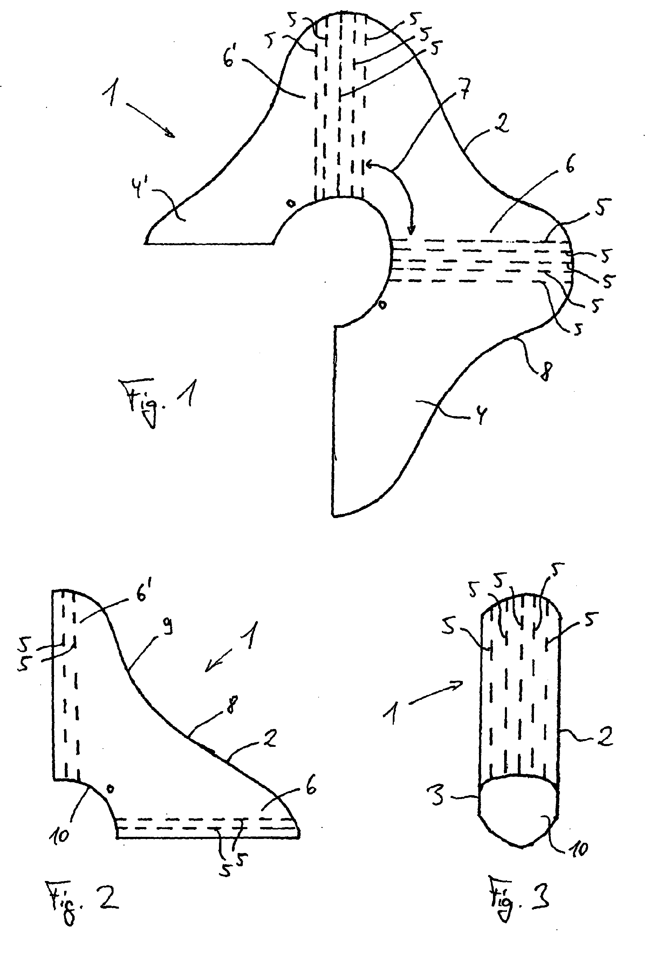

Fig. 1 stellt eine erfindungsgemäße Schiene 1 zur Ruhigstellung eines Gelenks dar, wobei ein Flächenkörper 2 im flächigen, nicht gefalteten Zustand dargestellt ist. Das dargestellte Flächenelement 2 ist durch Faltung um Falzlinien 5 der Falzliniengruppen 6, 6' zu einer Schlaufe faltbar, wobei die Endstücke 4, 4' im zusammengefalteten Zustand einander überlappen. Diese überlappenden Bereiche 4, 4' sind mittels Klebung oder Heftung miteinander verbindbar.

Die äußere Form, d.h. die Außenkontur 8, des Flächenkörpers 2 ist unregelmäßig, wobei die Ausbuchtungen den Teilen entsprechen, die mit den an ein Gelenk angrenzenden Körperteilen in Kontakt stehen, beispielsweise einem Unterarm und einem Oberarm oder einem Unterschenkel und einem Oberschenkel. In diesen ausgestülpten Bereichen sind auch die Gruppen 6, 6' von Falzlinien 5 angeordnet. In den Bereichen dazwischen ist die Außenkontur 8 weiter zurückgeführt, so dass Material eingespart wird, ohne die Festigkeit der Schiene 1 zu beeinträchtigen. Die Aussparung bietet den Platz für eine Befestigung der Schiene 1 mittels Umwicklung mit Verbandsmaterial.

Die Schiene 1 in Schlaufenform stabilisiert beispielsweise in einem Einsatz am Sprunggelenk den Fuß nach innen und außen (supination/pronation).

Die Gruppen 6, 6' von Falzlinien 5 sind unter einem Winkel 7 angeordnet, der in dem gezeigten Beispiel in Fig. 1 einem Winkel von 90° entspricht. Dies ist auch der Winkel, unter dem die angrenzenden Körperteile am Gelenk ruhig gestellt werden sollen. Der Winkel von 90° ist beispielsweise für ein Fußgelenk oder ein Ellenbogengelenk geeignet. Die Schiene ist allerdings auch mit Falzlinien unter anderen Winkeln als 90° einstellbar, abhängig von den Erfordernissen einer spezifischen Verletzung. So sind auch alle anderen Winkelstellungen möglich.

Im Zentrum des Flächenkörpers 2 ist eine Aussparung in Form eines Dreiviertelkreises erkennbar, die im zusammengefalteten Zustand der Schiene 1 eine kleine Öffnung bildet, durch die beispielsweise eine Ferse oder ein Ellenbogen passen. Auf diese Weise werden Teile der Gelenke, die nicht die gleiche Ebenmäßigkeit wie die angrenzenden Teile haben, beispielsweise ein Ellenbogen gegenüber einem Oberarm und einem Unterarm, nicht durch Material eingeengt.

Fig. 2 zeigt eine schematische Seitenansicht der erfindungsgemäßen Schiene 1 gemäß Fig. 1 im zusammengefalteten Zustand. Es ist die innere Öffnung bzw. kleine Öffnung 10 erkennbar, die am Ort des betroffenen Gelenks angeordnet ist. Gegenüber ist die große Öffnung 9 angeordnet, durch die beispielsweise ein Ellenbogen mit Oberarm und Unterarm in die Schiene eingeführt wird. Die Seitenwände des Flächenkörpers 2 bzw. der Schiene 1 sind größtenteils eben. Bei den verwendeten biegsamen Materialien wie Kunststoffplatten oder Wellpappe kann sich die Schiene 1 aber auch in den Seitenbereichen in begrenztem Maße an die Form des Gelenks und der anschließenden Körperteile anpassen, was den Tragekomfort erhöht.

Vorgeformte Falzlinien 5 sind in zwei Gruppen 6, 6' an den mit einem Oberarm und einem Unterarm in Kontakt stehenden Kanten vorgesehen. Die dargestellte Schiene 1 stellt einen Ellenbogen oder einen Fuß unter einem Winkel von 90° ruhig.

In Fig. 3 ist eine schematische Frontalansicht des Ausführungsbeispiels aus Fig. 1 und Fig. 2 gezeigt. Im unteren Bereich von Fig. 3 ist die kleine Öffnung 10 der Schlaufe 3 sichtbar, im oberen Teil die horizontale Rückwand mit den Falzlinien 5.

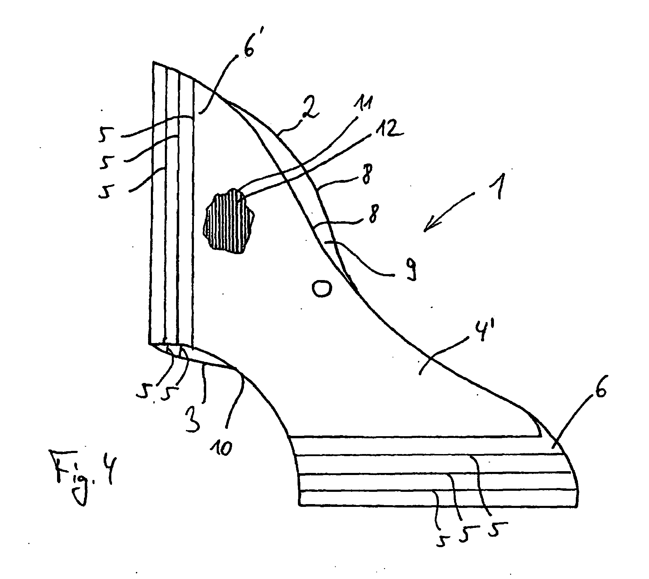

In Fig. 4 ist das Ausführungsbeispiel der Fig. 1 bis 3 in einer perspektivischen Ansicht schematisch dargestellt. Deutlich ist hier zu erkennen, wie die große Öffnung 9 und die kleine Öffnung 10 aufgebaut sind. An der Seite der Schiene 1 ist außerdem eine Einsicht in das Innere des Flächenkörpers 2 zur Verdeutlichung der Rippenstruktur 12 des aus Wellpappe bestehenden Flächenkörpers 2 gezeigt. Die Rippen 12 verlaufen dabei parallel zu den Falzlinien 5 der oberen Gruppe 6' von Falzlinien 5. An anderen Stellen ist es vorteilhaft, wenn die Rippenstruktur parallel zu der jeweils örtlich zu verstärkenden Richtung verläuft. Sie können auch schräg zu den Falzlinien 5 verlaufen, beispielsweise unter einem Winkel von 45°, so dass sich immer noch eine Verstärkung in der Beanspruchungsrichtung ergibt.

### Bezugszeichenliste

| | |
|---|---|
| 1 | Schiene |
| 2 | Flächenkörper |
| 3 | Schlaufe |
| 4, 4' | überlappende Bereiche |
| 5 | Falzlinien |
| 6, 6' | Gruppen von Falzlinien |
| 7 | Winkel zwischen Falzlinien |
| 8 | Außenkontur |
| 9 | große Öffnung |
| 10 | kleine Öffnung |
| 11 | Einsicht in das Innere des Flächenkörpers |
| 12 | Rippen von Wellpappe |

## Patentansprüche

1. Schiene (1) zur Ruhigstellung eines Gelenks, die am ruhig zu stellenden Gelenk zu tragen ist, wobei die Schiene (1) aus einem Flächenkörper (2) besteht, der zu einer geschlossenen Schlaufe (3) gebogen oder gefaltet ist, **dadurch gekennzeichnet, dass** die Außenkontur des Flächenkörpers (2) so ausgebildet ist, dass sich im zusammengesetzten Zustand eine Schlaufe (3) mit einer großen Öffnung (9) zur Aufnahme von Teilen der an das ruhig zu stellende Gelenk angrenzenden Körperteile und einer der großen Öffnung (9) gegenüber angeordneten kleinen Öffnung (10) am ruhig zu stellenden Gelenk ergibt.

2. Schiene (1) nach Anspruch 1, **dadurch gekennzeichnet, dass** zwei Bereiche (4, 4') des Flächenkörpers (2) überlappen, wobei die überlappenden Bereiche (4, 4') zur Bildung einer geschlossenen Schlaufe (3) fest miteinander verbunden oder verbindbar sind.

3. Schiene (1) nach Anspruch 2, **dadurch gekennzeichnet, dass** die feste Verbindung der überlappenden Bereiche (4, 4') eine Klebeverbindung, eine Steckverbindung oder eine Verbindung mittels Klammern, insbesondere Heftklammern, ist.

4. Schiene (1) nach einem der Ansprüche 1 bis 3, **dadurch gekennzeichnet, dass** der Flächenkörper (2) wenigstens zwei Gruppen (6, 6') von Falzlinien (5) mit jeweils wenigstens einer Falzlinie (5) aufweist, wobei die Falzlinien (5) jeder Gruppe (6, 6') untereinander parallel, aber zu den Falzlinien (5) der anderen Gruppen (6', 6) unter einem Winkel (7) angeordnet sind.

5. Schiene (1) nach Anspruch 4, **dadurch gekennzeichnet, dass** der Flächenkörper (2) durch Biegung um die Falzlinien (6) in die Form einer Schlaufe (3) bringbar ist.

6. Schiene (1) nach Anspruch 4 oder 5, **dadurch gekennzeichnet, dass** der Winkel (7) zwischen den Falzlinien (5) der verschiedenen Gruppen (6, 6') von Falzlinien (5) dem Einstellwinkel für das ruhig zu stellende Gelenk entspricht.

7. Schiene (1) nach einem der Ansprüche 1 bis 6, **dadurch gekennzeichnet, dass** die Schiene (1) in ihrer Kontur einem Schultergelenk, einem Ellenbogen, einem Kniegelenk oder einem Fußgelenk angepasst ist oder durch Zuschneiden anpassbar ist.

8. Schiene (1) nach einem der Ansprüche 1 bis 7, **dadurch gekennzeichnet, dass** der Flächenkörper (2) aus einem zugfesten Material besteht.

9. Schiene (1) nach einem der Ansprüche 1 bis 8, **dadurch gekennzeichnet, dass** der Flächenkörper (2) aus Pappe oder Karton besteht.

10. Schiene (1) nach Anspruch 9, **dadurch gekennzeichnet, dass** der Flächenkörper (2) aus Wellpappe besteht.

11. Schiene (1) nach Anspruch 10, **dadurch gekennzeichnet, dass** innen liegende Rippen (12) der Wellpappe wenigstens teilweise orthogonal zur Biegung des Flächenkörpers (2) oder wenigstens teilweise parallel zu den Falzlinien (5) des Flächenkörpers (2) ausgerichtet sind.

12. Schiene (1) nach einem der Ansprüche 1 bis 11, **dadurch gekennzeichnet, dass** die Schiene (1) symmetrisch zur Anwendung an einem Gelenk sowohl eines linken als auch eines rechten Körperteils ausgebildet ist.

## Claims

1. A splint (1) for immobilising a joint, which is to be carried on the joint to be immobilised, wherein the splint (1) consists of a flat body (2), which is bent or folded to form a closed loop (3), **characterised in that** the outer contour of the flat body (2) is so formed that, in the assembled state, a loop (3) is produced with a large opening (9) for receiving parts of the body portions adjoining the joint to be immobilised and a small opening (10), opposite to the large opening (9), at the joint to be immobilised.

2. A splint (1) as claimed in claim 1, **characterised in that** two regions (4, 4') of the flat body (2) overlap, wherein the overlapping regions (4, 4') are firmly connected or connectable together to form a closed loop (3).

3. A splint (1) as claimed in claim 2, **characterised in that** the firm connection of the overlapping regions (4, 4') is an adhesive connection, a plug connection or a connection by means of clips, particularly wire clips.

4. A splint (1) as claimed in claims 1 to 3, **characterised in that** the flat body (2) has at least two groups, (6, 6') of fold lines (5), each having at least one fold line (5), wherein the fold lines (5) of each group (6, 6') are parallel to one another but are disposed at an angle (7) to the fold lines (5) of the other groups (6', 6).

5. A splint (1) as claimed in claim 4, **characterised in that** the flat body (2) may be moved into the shape of a loop (3) by bending about the fold lines (6).

6. A splint (1) as claimed in claim 4 or 5, **characterised in that** the angle (7) between the fold lines (5) of the different groups (6, 6') of fold lines (5) corresponds to the setting angle for the joint to be immobilised.

7. A splint (1) as claimed in claims 1 to 6, **characterised in that** the contour of the splint is matched or is matchable by cutting to size to a shoulder joint, an elbow, a knee joint or a foot joint.

8. A splint (1) as claimed in claims 1 to 7, **characterised in that** the flat body (2) consists of a tension-resistant material.

9. A splint (1) as claimed in one of claims 1 to 8, **characterised in that** the flat body (2) consists of paperboard or cardboard.

10. A splint (1) as claimed in claim 9, **characterised in that** the flat body (2) consists of corrugated paperboard.

11. A splint (1) as claimed in claim 10, **characterised in that** internal ribs (12) of the corrugated paperboard are aligned at least partially orthogonally to the bending of the flat body (2) or at least partially parallel to the fold lines (5) of the flat body (2).

12. A splint (1) as claimed in one of claims 1 to 11, **characterised in that** the splint (1) is of symmetrical construction for use on a joint in both a left hand and also a right hand body portion.

## Revendications

1. Attelle (1) pour immobiliser une articulation, qui doit être portée sur l'articulation à immobiliser, sachant que l'attelle (1) est constituée d'un corps plan (2) qui est recourbé ou replié en une boucle fermée (3), **caractérisée en ce que** le contour extérieur du corps plan (2) est configuré de telle sorte qu'on obtient, dans l'état monté, une boucle (3) avec une grande ouverture (9), destinée à recevoir des parties des membres adjacents à l'articulation à immobiliser, et une petite ouverture (10), disposée en vis-à-vis de la grande ouverture (9) et située sur l'articulation à immobiliser.

2. Attelle (1) selon la revendication 1, **caractérisée en ce que** deux régions (4, 4') du corps plan (2) se recouvrent, les régions en recouvrement (4, 4') étant ou pouvant être fixement assemblées entre elles afin de former une boucle fermée (3).

3. Attelle (1) selon la revendication 2, **caractérisée en ce que** l'assemblage fixe entre les régions en recouvrement (4, 4') est un assemblage collé, un assemblage par emboîtement ou un assemblage au moyen d'attaches, notamment d'agrafes.

4. Attelle (1) selon l'une des revendications 1 à 3, **caractérisée en ce que** le corps plan (2) présente au moins deux groupes (6, 6') de lignes de pliage (5) comportant chacun au moins une ligne de pliage (5), sachant que les lignes de pliage (5) de chaque groupe (6, 6') sont disposées parallèles entre elles, mais sous un angle (7) par rapport aux lignes de pliage (5) des autres groupes (6', 6).

5. Attelle (1) selon la revendication 4, **caractérisée en ce que** le corps plan (2) peut être amené sous la forme d'une boucle (3) par courbure autour des lignes de pliage (5).

6. Attelle (1) selon la revendication 4 ou 5, **caractérisée en ce que** l'angle (7) entre les lignes de pliage (5) des différents groupes (6, 6') de lignes de pliage (5) correspond à l'angle de réglage pour l'articulation à immobiliser.

7. Attelle (1) selon l'une des revendications 1 à 6, **caractérisée en ce que** le contour de l'attelle (1) est adapté, ou peut être adapté par découpage, à l'articulation de l'épaule, à l'articulation du coude, à l'articulation du genou ou à l'articulation du pied.

8. Attelle (1) selon l'une des revendications 1 à 7, **caractérisée en ce que** le corps plan (2) est constitué d'un matériau résistant à la traction.

9. Attelle (1) selon l'une des revendications 1 à 8, **caractérisée en ce que** le corps plan (2) est constitué de carton ou de carton fort.

10. Attelle (1) selon la revendication 9, **caractérisée en ce que** le corps plan (2) est constitué de carton ondulé.

11. Attelle (1) selon la revendication 10, **caractérisée en ce que** des nervures intérieures (12) du carton ondulé sont orientées au moins pour partie orthogonalement à la courbure du corps plan (2) ou au moins pour partie parallèlement aux lignes de pliage (5) du corps plan (2).

12. Attelle (1) selon l'une des revendications 1 à 11, **caractérisée en ce que** l'attelle (1) est réalisée symétrique, en vue d'être utilisée sur une articulation tant d'un membre gauche que d'un membre droit.
